(19) Europäisches Patentamt
European Patent Office
Office européen des brevets

(11) Numéro de publication: **0 165 124**
**B1**

# (12) FASCICULE DE BREVET EUROPEEN

(45) Date de publication du fascicule du brevet: **13.07.88**

(21) Numéro de dépôt: **85400922.2**

(22) Date de dépôt: **13.05.85**

(51) Int. Cl.⁴: **C 07 C 51/00**, C 07 C 59/64, C 07 C 57/30, C 07 C 57/58, C 07 C 67/475, C 07 C 69/612, C 07 C 69/734

## (54) Procédé de préparation d'acides hydratropiques et de leurs esters, à partir de propiophénones.

(30) Priorité: **16.05.84 FR 8407544**

(43) Date de publication de la demande: **18.12.85 Bulletin 85/51**

(45) Mention de la délivrance du brevet: **13.07.88 Bulletin 88/28**

(84) Etats contractants désignés: **AT BE CH DE GB IT LI NL SE**

(56) Documents cités: **EP - A - 0 071 299**

(73) Titulaire: **DELALANDE S.A., 32, rue Henri Regnault, F-92400 Courbevoie (FR)**

(72) Inventeur: **Adrian, Guy, 56, rue de Dobeln, F-69700 Givors (FR)**

(74) Mandataire: **Kedinger, Jean-Paul et al, c/o Cabinet Malemont 42, avenue du Président Wilson, F-75116 Paris (FR)**

EP 0 165 124 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau procédé de préparation d'acides hydratropiques et de leurs esters, répondant à la formule:

$$Ar{-}\underset{\displaystyle CH_3}{\underset{|}{C}}H{-}COOR \qquad (I)$$

dans laquelle:

– R représente un atome d'hydrogène ou un groupe alkyle inférieur, et

– Ar représente un noyau phényle ou un noyau phényle substitué par au moins un groupe non susceptible de désactiver le noyau phényle.

Un procédé de préparation de tels composés est déjà décrit dans la demande de brevet européen No. 034 871 où il est proposé d'opérer en trois étapes successives, à savoir:

a) réaction du brome sur une propiophénone de formule:

$$Ar^{o}{-}\underset{\displaystyle O}{\underset{||}{C}}{-}CH_2CH_3$$

où $Ar^{o}$ est un noyau aryle,

b) réaction de la bromopropiophénone résultante avec un orthoester de formule:

$$R^{o}_1{=}C(OR^{o})_3$$

où $R^{o}_1{=}H$ et $R^{o}$ = alkyle inférieur et un alcool primaire de formule:

$$R^{o}{-}OH$$

où $R^{o}$ = alkyle inférieur

c) transposition de l'acétal résultant en présence d'un sel métallique organique ou minéral.

La difficulté principale de cette voie de synthèse réside dans l'étape d'acétalisation b) qui est une réaction équilibrée nécessitant un excès de réactifs d'acétalisation et des temps de reflux prolongés pour un rendement de 70–80% seulement. La productivité est donc faible au niveau de cette étape b) et la voie de synthèse proposée dans le document ci-dessus demande de ce fait à être améliorée en vue d'une exploitation industrielle rentable.

La demanderesse a été amenée à étudier de nouvelles voies de synthèse des acides hydratropiques (composés à activité antiinflammatoire) et des esters correspondants, voies de synthèse susceptibles de conduire de manière plus économiques auxdits acides et esters. Elle a ainsi trouvé que les composés de formule (I) pouvaient être obtenus avec d'excellents rendements et sans consommation excessive de réactifs, en faisant réagir sur les propiophénones de formule:

$$Ar{-}\underset{\displaystyle O}{\underset{||}{C}}{-}CH_2CH_3 \qquad (II)$$

où Ar a la même signification que dans la formule (I), un agent chlorant ou bromant, un alcool primaire de formule:

$$R'{-}OH \qquad (III)$$

où R' représente un groupe alkyle inférieur, un orthoester de formule:

$$R_1{-}C(OR')_3 \qquad (IV)$$

où $R_1$ représente un atome d'hydrogène ou un groupe alkyle inférieur et R' a la même signification que dans la formule (III), et du zinc métallique, de préférence en poudre, puis en hydrolysant éventuellement les esters résultants de formule:

$$Ar{-}\underset{\displaystyle CH_3}{\underset{|}{C}}H{-}COOR' \qquad (Ia)$$

où R' a la même signification que dans la formule (III) ou (IV), pour obtenir les acides correspondants de formule:

$$Ar{-}\underset{\displaystyle CH_3}{\underset{|}{C}}H{-}COOH \qquad (Ib)$$

Conformément à l'invention, on peut d'abord faire réagir l'agent chlorant ou bromant sur les propiophénones (II), puis faire réagir sur les composants résultants (chloropropiophénones ou bromopropiophénones), simultanément l'alcool (III), l'orthoester (IV) et le zinc métallique. On peut en variante faire réagir sur les propiophénones (II), simultanément l'agent chlorant ou bromant, l'alcool (III), l'orthoester (IV) et le zinc métallique, ce qui peut par exemple être réalisé en ajoutant l'agent chlorant ou bromant progressivement au mélange propiophénone (II) / alcool (III) / orthoester (IV) / zinc métallique.

Il est à noter que la réaction simultanée de l'agent chlorant ou bromant, de l'alcool (III), de l'orthoester (IV) et du zinc métallique sur les propiophénones (II) ou la réaction simultanée de l'alcool (III), de l'orthoester (IV) et du zinc métallique sur le produit de réaction de l'agent chlorant ou bromant et des propiophénones (II) devra être effectuée à chaud, de préférence à une température de 80–130 °C. On pourra par exemple opérer d'abord à une température modérée (notamment jusqu'à 45 °C environ) puis élever progressivement la température du milieu réactionnel jusqu'à la temperature finale desirée en éliminant, au cours de cette élévation de température, les produits volatils ayant une température d'ébullition inférieur à ladite température finale désirée.

Dans l'état actuel des connaisances du mécanisme réactionnel, il semble que les propiophénones (II) réagissent d'abord avec l'agent chlorant ou bromant pour conduire aux chloro- ou brompropiophénones correspondantes qui entrent à leur tour en réaction avec le zinc métallique pour former des composés organozinciques qui évoluent ensuite à chaud jusqu'aux composés (Ia)

suivant un processus ne faisant intervenir aucune réaction équilibrée susceptible de porter préjudice aux rendements de la réaction.

Le procédé selon l'invention permet ainsi, en une ou deux étape(s), d'obtenir les esters (Ia) recherchés avec des rendements pouvant atteindre 95% et ce, avec des durées de réaction de l'ordre de 2 à 6 heures seulement.

Conformément à l'invention, on utilisera avantageusement un agent bromant qui est généralement plus réactif qu'un agent chlorant. A titre d'agent bromant, on citera par exemple le brome, le brom-chlore ou le perbromure de triméthylphényl ammonium, le brome étant particulièrement préféré en raison de sa disponibilité, de sa bonne réactivité et de son point d'ébullition qui permet de la faire réagir à une température relativement élevée.

On pourra, si cela est nécessaire, opérer en présence d'un diluant inerte dans la réaction considérée, apte à dissoudre les composés (II), (III) et (IV et possédant un point d'ébullition permettant d'amener le mélange réactionnel à la température désirée; il pourra s'agir notamment de diluants halogénés tels que le tétrachloroéthylène, le tétrachloroéthane ou le chlorobenzéne.

La quantité de zinc métallique à mettre en œuvre n'est pas critique et pour des questions évidentes d'économie et de facilité de séparation du mélange de réaction final, on l'utilisera de préférence en quantité catalytique.

L'agent chlorant ou bromant et l'orthoester (IV) seront, pour leur part, utilisés respectivement à raison d'au moins un équivalent molaire et d'au moins deux équivalents molaires par rapport à la propiophénone (II).

Quant à l'alcool primaire (III), il sera utilisé à raison d'au moins un équivalent molaire par rapport à la propiophénone (II); on préfère cependant l'utiliser en large excès auquel cas il jouera simultanément le rôle de diluant.

L'hydrolyse des esters (Ia) pourra être realisée en faisant appel aux techniques classiques d'hydrolyse acide ou alcaline d'un ester en l'acide correspondant, tels que par exemple traitement des esters (Ia) par des alcalis aqueux (notamment soude aqueuse) dans un solvant (notamment alcool inférieur), suivi d'une acidification.

Parmi les composés (I) pouvant être préparés conformément au procédé selon l'invention, on citera notamment ceux pour lesquels R=H ou un groupe alkyle inférieur normal tel que méthyle, éthyle, n-propyle et n-butyle et Ar est choisi dans le groupe comprenant:

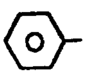 , 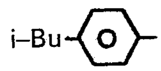 , 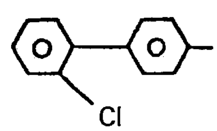 , 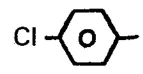 , 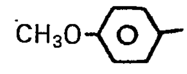

On citera en particulier le composé (I) de structure particulière:

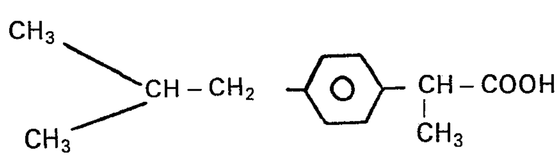

qui est l'ibuprofène, agent antiinflammatoire notoirement connu et les esters correspondants, précurseurs de l'ibuprofène.

Il est à noter enfin que parmi les composés (I), celui de structure particulière:

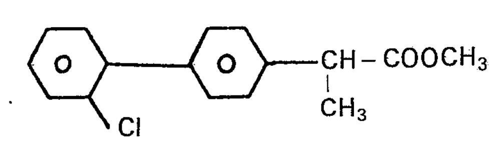 (I'a)

est nouveau; l'invention s'étend par conséquent à ce nouveau composé.

On donnera ci-après à titre d'exemples, la préparation de quelques composés (I) pour illustrer l'invention.

Exemple 1

(chloro-2' biphénylyl-4)-2 propionate de méthyle

Dans un tricol de 1 litre, on place:

- 121 g (0,494 mole) de (chloro-2' biphénylyl-4)-1 propanone-1,
- 130 g (1,23 mole) de triméthyl orthoformiate,
- 242 ml de méthanol, et
- 2,6 g (0,04 at.g) de zinc en poudre.

On porte le mélange à 45 °C sous agitation et y ajoute en 20 minutes 83 g (0,52 mole) de brome. Le milieu réactionnel devient jaune paille. On porte progressivement en 1 heure le milieu de 45 °C à 115 °C en distillant les produits volatils entre 32 et 65 °C. On maintient pendant 45 minutes entre 115 et 120 °C, puis refroidit à 30 °C et ajoute 250 ml d'eau. On extrait le milieu réactionnel à deux reprises avec 100 ml de chlorure de méthylène. On concentre la phase organique puis la distille sous vide, ce qui conduit à l'obtention de 111 g du produit attendu.

- Rendement: 81%
- $E_{0,1 torr}$ 155 °C
- Point de fusion: 63 °C (cristaux blancs)
- Spectre IR (KBr): ν C=O 1740 $cm^{-1}$
- Spectre RMN ($CDCl_3$) δ ppm:

1,6 (d, 3H, J=7Hz, $CH_3$); 3,7 (s, 3H, $OCH_3$); 3,8 (q, 1H, J=7Hz, CH); 7,3–7,7 (m, 8H aromatiques).

Exemple 2

phényl-2 propionate d'éthyle

Dans un tricol de 0,5 litre, on place:

- 26,8 g (0,2 mole) de propiophénone,

- 74 g (0,5 mole) de triéthyl orthoformiate,
- 60 ml d'éthanol, et
- 0,43 g (0,016 at.g) de zinc en poudre.

On ajoute à 45 °C et en 20 minutes 33,5 g (0,21 mole) brome puis distille entre 30 et 80 °C les produits volatils en chauffant le milieu de 45 à 120 °C. On maintient cette dernière température pendant 1 heure. Le milieu est ensuite dilué par 100 ml de toluène, puis lavé par 250 ml d'eau. Par distillation, on isole 30 g du composé attendu.

- Rendement: 84%
- $E_{10\,torrs}$ 95 °C
- $n_D^{20}$: 1,5025 (en accord avec la littérature)
- Pureté: 98%

Exemple 3

(isobutyl-4 phényl)-2 propionate de méthyle

Dans un réacteur de 1 litre, on intruit:

- 190,3 g (1 mole) de para-isobutylpropiophénone,
- 220 g (2,08 moles) de triméthyl-orthoformiate,
- 380 ml de méthanol, et
- 5,2 g (0,08 at.g) de zinc en poudre.

On porte le mélange sous agitation à 45 °C et on ajoute en 20 minutes 162 g (1,013 mole) de brome. On porte progressivement en 1 heure le milieu de 45 à 115 °C en distillant entre 32 et 65 °C. On maintient 1 heure à 115 °C, refroidit à 30 °C, ajoute 500 ml d'eau, extrait deux fois par 200 ml de chlorure de méthylène. On concentre la phase organique et la distille sous vide. On isole ainsi 197 g du composé attendu.

- Rendement: 89%
- $E_{0,1\,torr}$: 95 °C
- Titre CGL: 99,5%

Exemple 4

acide (isobutyl-4 phényl)-2 propionique ou ibuprofène

Dans un réacteur de 6 litres, on charge:

- 749 g (3,95 moles) de para-isobutylpropiophénone,
- 951 g (8,96 moles) de triméthyl orthoformiate,
- 1500 ml de méthanol,
- 20,9 g (0,32 at.g) de zinc en poudre, et
- 750 ml de tétrachloroéthylène utilisé comme diluant.

On chauffe à 40 °C en agitant et on ajoute en 1 heure 656 g (4,1 moles) de brome. On maintient le mélange pendant 30 minutes à 45 °C puis distille 2 litres de produits de point d'ébullition compris entre 34 et 62 °C, la température interne étant comprise entre 45 et 85 °C. A ce stade, on rajoute 750 ml de tétrachloroéthylène et porte à 100 °C. Une vigoureuse ébullition se produit et la température interne se stabilise à 128 °C. Après 1 heure on refroidit à 75 °C et ajoute 2 litres d'eau. On décante et concentre la phase organique à 80 °C sous 20 torrs pour récupérer 1400 ml de tétrachloroéthylène et 886 g d'un résidu constitué principalement de para-isobutylphényl-2 propionate de méthyle. Ce produit brut est dissous dans 2,25 litres de méthanol mélangés avec 400 g (10 moles) de soude et 2,5 litres d'eau. On porte à reflux pendant 2 heures, distille le méthanol jusqu'à atteindre une température interne de 95 °C en recueillant 2,5 litres d'un mélange méthanol-eau. On laisse cristalliser 6 heures et filtre le sel de sodium formé. On le lave sur le filtre avec 1 litre de tétrachloroéthylène, on l'essore et le redissout dans 2 litres d'eau. Puis on acidifie à pH 2 par 390 ml d'acide chlorhydrique à 36%, laisse cristalliser à 15 °C, filtre le précipité formé, essore ce dernier et le sèche à 50 °C, ce qui conduit à 670 g (rendement: 81%) du produit attendu (Point de fusion: 76 °C).

**Revendications pour les Etats contractants: DE, NL, GB, CH, BE, SE, IT)**

1. Procédé de préparation d'acides hydratropiques et de leurs esters, répondant à la formule:

$$Ar{-}\underset{\displaystyle CH_3}{\underset{|}{C}H}{-}COOR \qquad (I)$$

dans laquelle:

– R représente un atome d'hydrogène ou un groupe alkyle inférieur, et

– Ar représente un noyau phényle ou un noyau phényle substitué par au moins un groupe non susceptible de désactiver le noyau phényle, caractérisé en ce qu'il consiste à faire réagir sur les propiophénones de formule:

$$Ar{-}\underset{\displaystyle O}{\underset{\|}{C}}{-}CH_2CH_3 \qquad (II)$$

où Ar a la même signification que dans la formule (I), un agent chlorant ou bromant, un alcool primaire de formule:

$$R'{-}OH \qquad (III)$$

où R' représente un groupe alkyle inférieur, un orthoester de formule:

$$R_1{-}C(OR')_3 \qquad (IV)$$

où $R_1$ représente un atome d'hydrogène ou un groupe alkyle inférieur et R' a la même signification que dans la formule (III), et du zinc métallique, puis éventuellement à hydrolyser les esters résultants de formule:

$$Ar{-}\underset{\displaystyle CH_3}{\underset{|}{C}H}{-}COOR' \qquad (Ia)$$

où R' a la même signification que dans la formule (III) ou (IV), pour obtenir les acides correspondants de formule:

$$Ar{-}\underset{\displaystyle CH_3}{\underset{|}{C}H}{-}COOH \qquad (Ib)$$

2. Procédé selon la revendication 1, caractérisé en ce qu'il consiste à faire réagir l'agent chlorant ou bromant sur les propiophénones (II), puis à faire réagir sur les composés résultants, simultanément l'alcool (III), l'orthoester (IV) et le zinc métallique.

3. Procédé selon la revendication 1, caractérisé en ce qu'il consiste à faire réagir simultanément sur les propiophénones (II), l'agent chlorant ou bromant, l'alcool (III), l'orthoester (IV) et le zinc métallique.

4. Procédé selon la revendication 1, 2 ou 3, caractérisé en ce que la réaction simultanée de l'agent chlorant ou bromant, de l'alcool (III), de l'orthoester (IV) et du zinc métallique sur les propiophénones (II) ou la réaction simultanée de l'alcool (III), de l'orthoester (IV) et du zinc métallique sur le produit de réaction de l'agent chlorant ou bromant et des propiophénones (II), est effectuée à chaud.

5. Procédé selon la revendication 4, caractérisé en ce que ladite réaction est effectuée à 80–130 °C.

6. Procédé selon l'une quelconque des revendications précédentes dans lequel on met en œuvre un agent bromant, caractérisé en ce que ce dernier est le brome.

7. Procédé selon l'une quelconque des revendications précédentes, caractérisé en ce que le zinc métallique est utilisé en quantité catalytique.

8. Procédé selon l'une quelconque des revendications précédentes, caractérisé en ce que l'agent chlorant ou bromant et l'orthoester (IV) sont utilisés respectivement à raison d'au moins un équivalent molaire et d'au moins deux équivalents molaires par rapport à la propiophénone (II), l'alcool (III) étant utilisé à raison d'au moins un équivalent par rapport à la propiophénone (II).

9. Procédé selon l'une quelconque des revendications précédentes, caractérisé en ce que dans les formules (I) à (IV), R et R' représentent un groupe alkyle inférieur normal et Ar est un groupe choisi parmi les suivants:

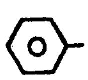 , 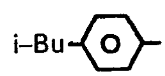 , 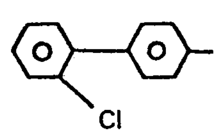 , 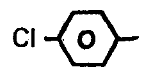 , 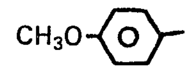

10. A titre de produit industriel nouveau, le composé de formule:

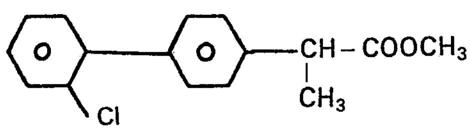 (I'a)

## Revendications pour l'Etat contractant: AT

1. Procédé de préparation d'acides hydratropiques et de leurs esters, répondant à la formule:

$$Ar\text{-}\underset{\displaystyle CH_3}{\underset{|}{C}H}\text{-}COOR \qquad (I)$$

dans laquelle:

– R représente un atome d'hydrogène ou un groupe alkyle inférieur, et

– Ar représente un noyau phényle ou un noyau phényle substitué par au moins un groupe non susceptible de désactiver le noyau phényle, caractérisé en ce qu'il consiste à faire réagir sur les propiophénones de formule:

$$Ar\text{-}\underset{\displaystyle O}{\underset{||}{C}}\text{-}CH_2CH_3 \qquad (II)$$

où Ar a la même signification que dans la formule (I), un agent chlorant ou bromant, un alcool primaire de formule:

$$R'\text{-}OH \qquad (III)$$

où R' représente un groupe alkyle inférieur, un orthoester de formule:

$$R_1\text{-}C(OR')_3 \qquad (IV)$$

où $R_1$ représente un atome d'hydrogène ou un groupe alkyle inférieur et R' a la même signification que dans la formule (III) et du zinc métallique, puis éventuellement à hydrolyser les esters résultants de formule:

$$Ar\text{-}\underset{\displaystyle CH_3}{\underset{|}{C}H}\text{-}COOR' \qquad (Ia)$$

où R' a la même signification que dans la formule (III) ou (IV), pour obtenir les acides correspondants de formule:

$$Ar\text{-}\underset{\displaystyle CH_3}{\underset{|}{C}H}\text{-}COOH \qquad (Ib)$$

2. Procédé selon la revendication 1, caractérisé en ce qu'il consiste à faire réagir l'agent chlorant ou bromant sur les propiophénones (II), puis à faire réagir sur les composés resultants, simultanément l'alcool (III), l'orthoester (IV) et le zinc métallique.

3. Procédé selon la revendication 1, caractérisé en ce qu'il consiste à faire réagir simultanément sur les propiophénones (II), l'agent chlorant ou bromant, l'alcool (III), l'orthoester (IV) et le zinc métallique.

4. Procédé selon la revendication 1, 2 ou 3, caractérisé en ce que la réaction simultanée de l'agent chlorant ou bromant, de l'alcool (III), de l'orthoester (IV) et du zinc métallique sur les propiophénones (II) ou la réaction simultanée de l'alcool (III), de l'orthoester (IV) et du zinc métallique sur le produit de réaction de l'agent chlorant ou bromant et des propiophénones (II), est effectuée à chaud.

5. Procédé selon la revendication 4, caractérisé en ce que ladite réaction est effectuée à 80–130 °C.

6. Procédé selon l'une quelconque des revendications précédentes dans lequel on met en œuvre un agent bromant, caractérisé en ce que ce dernier est le brome.

7. Procédé selon l'une quelconque des revendications précédentes, caractérisé en ce que le zinc métallique est utilisé en quantité catalytique.

8. Procédé selon l'une quelconque des revendications précédentes, caractérisé en ce que l'agent chlorant ou bromant et l'orthoester (IV) sont utilisés respectivement à raison d'au moins un équivalent molaire et d'au moins deux équivalents molaires par rapport à la propiophénone (II), l'alcool (III) étant utilisé à raison d'au moins un équivalent par rapport à la propiophénone (II).

9. Procédé selon l'une quelconque des revendications précédentes, caractérisé en ce que dans les formules (I) à (IV), R et R' représentent un groupe alkyle inférieur normal et Ar est un groupe choisi parmi les suivants:

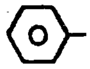 , 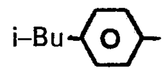 , 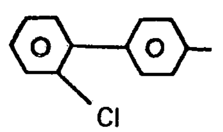 , 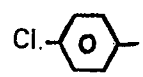 , 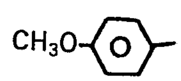

**Patentansprüche für die Vertragsstaaten DE, NL, GB, CH, BE, SE, IT**

1. Verfahren zur Herstellung von Hydratropasäuren und ihren Estern, die der Formel entsprechen:

$$Ar{-}\underset{CH_3}{\underset{|}{CH}}{-}COOR \qquad (I)$$

worin bedeuten:

– R ein Wasserstoffatom oder eine niedere Alkylgruppe und

– Ar einen Phenylkern oder einen Phenylkern, der durch mindestens eine Gruppe substituiert ist, die den Phenylkern nicht desaktivieren kann, dadurch gekennzeichnet, dass es darin besteht, dass man auf die Propiophenone der Formel

$$Ar{-}\underset{O}{\underset{||}{C}}{-}CH_2{-}CH_3 \qquad (II)$$

worin Ar die gleiche Bedeutung wie in der Formel (I) hat, ein Chlorierungsmittel oder ein Bromierungsmittel, einen primären Alkohol der Formel

$$R'{-}OH \qquad (III)$$

worin R' eine niedere Alkylgruppe bedeutet, einen Orthoester der Formel

$$R_1{-}C(OR')_3 \qquad (IV)$$

worin $R_1$ ein Wasserstoffatom oder eine niedere Alkylgruppe darstellt und R' die gleiche Bedeutung wie in der Formel (III) hat, und metallisches Zink einwirken lässt und dann ggf. die resultierenden Ester der Formel

$$Ar{-}\underset{CH_3}{\underset{|}{CH}}{-}COOR' \qquad (Ia)$$

worin R' die gleiche Bedeutung wie in der Formel (III) oder (IV), hat, hydrolysiert unter Bildung der entsprechenden Säuren der Formel

$$Ar{-}\underset{CH_3}{\underset{|}{CH}}{-}COOH \qquad (Ib)$$

2. Verfahren nach Anspruch 1, dadurch gekennzeichnet, dass es darin besteht, dass man das Chlorierungsmittel oder Bromierungsmittel auf die Propiophenone (II) einwirken lässt, dann auf die resultierenden Verbindungen gleichzeitig den Alkohol (III), den Orthoester (IV) und das metallische Zink einwirken lässt.

3. Verfahren nach Anspruch 1, dadurch gekennzeichnet, dass es darin besteht, dass man gleichzeitig auf die Propiophenone (II) das Chlorierungs- oder Bromierungsmittel, den Alkohol (III), den Orthoester (IV) und das metallische Zink einwirken lässt.

4. Verfahren nach Anspruch 1, 2 oder 3, dadurch gekennzeichnet, dass man gleichzeitig das Chlorierungs- oder Bromierungsmittel, den Alkohol (III), den Orthoester (IV) und das metallische Zink mit den Propiophenonen (II) in der Wärme reagieren lässt oder dass man gleichzeitig den Alkohol (III), den Orthoester (IV) und das metallische Zink mit dem Produkt der Umsetzung zwischen dem Chlorierungs- oder Bromierungsmittel und den Propiophenonen (II) in der Wärme reagieren lässt.

5. Verfahren nach Anspruch 4, dadurch gekennzeichnet, dass man die Reaktion bei 80 bis 130 °C durchführt.

6. Verfahren nach einem der vorhergehenden Ansprüche, bei dem man ein Bromierungsmittel einsetzt, dadurch gekennzeichnet, dass es sich bei letzterem um Brom handelt.

7. Verfahren nach einem der vorhergehenden Ansprüche, dadurch gekennzeichnet, dass das metallische Zink in einer katalytischen Menge verwendet wird.

8. Verfahren nach einem der vorhergehenden Ansprüche, dadurch gekennzeichnet, dass das Chlorierungs- oder Bromierungsmittel und der Orthoester (IV) jeweils in einer Menge von mindestens einem Moläquivalent und mindestens 2 Moläquivalenten, bezogen auf das Propiophenon (II), verwendet werden, dass der Alkohol (III) in einer Menge von mindestens einem Äquivalent, bezogen auf das Propiophenon (II), verwendet wird.

9. Verfahren nach einem der vorhergehenden Ansprüche, dadurch gekennzeichnet, dass in den Formeln (I) bis (IV) R und R' eine normale niedere Alkylgruppe und Ar eine Gruppe darstellen, die unter den folgenden ausgewählt wird:

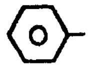, 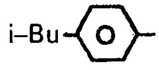, 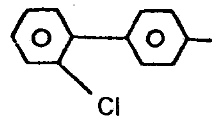, 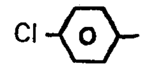, 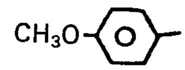

10. Als neues industrielles Produkt die Verbindung der Formel

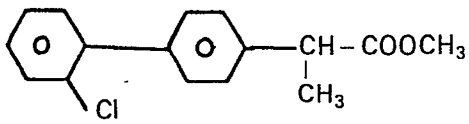 (I'a)

**Patentansprüche für den Vertragsstaat AT**

1. Verfahren zur Herstellung von Hydratropasäuren und ihren Estern, die der Formel entsprechen:

$$Ar-\underset{\displaystyle CH_3}{\underset{|}{C}H}-COOR \qquad (I)$$

worin bedeuten:

– R ein Wasserstoffatom oder eine niedere Alkylgruppe und

– Ar einen Phenylkern oder einen Phenylkern, der durch mindestens eine Gruppe substituiert ist, die den Phenylkern nicht desaktivieren kann, dadurch gekennzeichnet, dass es darin besteht, dass man auf die Propiophenone der Formel

$$Ar-\underset{\displaystyle O}{\underset{\|}{C}}-CH_2-CH_3 \qquad (II)$$

worin Ar die gleiche Bedeutung wie in der Formel (I) hat, ein Chlorierungs- oder Bromierungsmittel, einen primären Alkohol der Formel

$$R'-OH \qquad (III)$$

worin R' eine niedere Alkylgruppe bedeutet, einen Orthoester der Formel

$$R_1-C(OR')_3 \qquad (IV)$$

worin $R_1$ ein Wasserstoffatom oder eine niedere Alkylgruppe darstellt und R' die gleiche Bedeutung wie in der Formel (III) hat, und metallisches Zink einwirken lässt und dann ggf. die resultierenden Ester der Formel

$$Ar-\underset{\displaystyle CH_3}{\underset{|}{C}H}-COOR' \qquad (Ia)$$

worin R' die gleiche Bedeutung wie in der Formel (III) oder (IV) hat, hydrolysiert zur Herstellung der entsprechenden Säuren der Formel

$$Ar-\underset{\displaystyle CH_3}{\underset{|}{C}H}-COOH \qquad (Ib)$$

2. Verfahren nach Anspruch 1, dadurch gekennzeichnet, dass es darin besteht, dass man das Chlorierungs- oder Bromierungsmittel auf die Propiophenone (II) einwirken lässt, dass man dann auf die resultierenden Verbindungen gleichzeitig den Alkohol (III), den Orthoester (IV) und das metallische Zink einwirken lässt.

3. Verfahren nach Anspruch 1, dadurch gekennzeichnet, dass es darin besteht, dass man auf die Propiophenone gleichzeitig das Chlorierungs- oder Bromierungsmittel, den Alkohol (III), den Orthoester (IV) und das metallische Zink einwirken lässt.

4. Verfahren nach Anspruch 1, 2 oder 3, dadurch gekennzeichnet, dass man gleichzeitig das Chlorierungs- oder Bromierungsmittel, den Alkohol (III), den Orthoester (IV) und das metallische Zink mit den Propiophenonen (II) in der Wärme reagieren lässt oder dass man gleichzeitig den Alkohol (III), den Orthoester (IV) und das metallische Zink mit dem Produkt der Umsetzung zwischen dem Chlorierungs- oder Bromierungsmittel und den Propiophenonen (II) in der Wärme reagieren lässt.

5. Verfahren nach Anspruch 4, dadurch gekennzeichnet, dass man die Reaktion bei 80 bis 130 °C durchführt.

6. Verfahren nach einem der vorhergehenden Ansprüche, bei dem man ein Bromierungsmittel einsetzt, dadurch gekennzeichnet, dass es sich bei letzterem um Brom handelt.

7. Verfahren nach einem der vorhergehenden Ansprüche, dadurch gekennzeichnet, dass man das metallische Zink in katalytischer Menge verwendet.

8. Verfahren nach einem der vorhergehenden Ansprüche, dadurch gekennzeichnet, dass man das Chlorierungs- oder Bromierungsmittel und den Orthoester (IV) jeweils in einer Menge von mindestens einem Moläquivalent und mindestens 2 Moläquivalenten, bezogen auf das Propiophenon (II), verwendet, dass man den Alkohol (III) in einer Menge von mindestens einem Äquivalent, bezogen auf das Propiophenon (II), verwendet.

9. Verfahren nach einem der vorhergehenden Ansprüche, dadurch gekennzeichnet, dass in den Formeln (I) bis (IV) R und R' eine normale niedere Alkylgruppe und Ar eine Gruppe darstellen, die unter den folgenden ausgewählt wird:

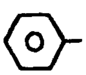, 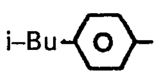, 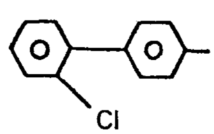, 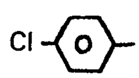, 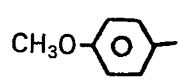

**Claims for the contracting states: DE, NL, GB, CH, BE, SE, IT**

1. A process for preparing hydratropic acids and the esters thereof, corresponding to the formula:

$$Ar{-}\underset{\displaystyle CH_3}{\underset{|}{CH}}{-}COOR \qquad (I)$$

in which:

– R represents a hydrogen atom or a lower alkyl group, and

– Ar represents a phenyl nucleus or a phenyl nucleus substituted by at least one group not likely to de-activate the phenyl nucleus, characterized in that it consists in reacting on the propiophenones of formula:

$$Ar{-}\underset{\displaystyle O}{\underset{\|}{C}}{-}CH_2CH_3 \qquad (II)$$

wherein Ar has the same meaning as in formula (I), a chlorinating or brominating agent, a primary alcohol of formula:

$$R'{-}OH \qquad (III)$$

wherein R′ represents a lower alkyl group, an orthoester of formula:

$$R_1{-}C(OR')_3 \qquad (IV)$$

wherein $R_1$ represents a hydrogen atom or a lower alkyl group and R′ has the same meaning as in formula (III) and metal zinc, then possibly hydrolyzing the resulting esters of formula:

$$Ar{-}\underset{\displaystyle CH_3}{\underset{|}{CH}}{-}COOR' \qquad (Ia)$$

wherein R′ has the same meaning as in formula (III) or (IV), to obtain the corresponding acids of formula:

$$Ar{-}\underset{\displaystyle CH_3}{\underset{|}{CH}}{-}COOH \qquad (Ib)$$

2. The process as claimed in claim 1, characterized in that it consists in reacting the chlorinating or brominating agent on the propiophenons (II), then in reacting on the resulting compounds, simultaneously the alcohol (III), the orthoester (IV) and the metal zinc.

3. The process as claimed in claim 1, characterized in that it consists in reacting simultaneously on the propiophenones (II), the chlorinating or brominating agent, the alcohol (II), the orthoester (IV) and the metal zinc.

4. The process as claimed in claim 1, 2 or 3, characterized in that the simultaneous reaction of the chlorinating or brominating agent, of the alcohol (III), of the orthoester (IV) and of the metal zinc on the propiophenones (II) or the simultaneous reaction of the alcohol (III), of the orthoester (IV) and of the metal zinc on the reaction product of the chlorinating or bromating agent and the propiophenones is carried out under heating.

5. The process as claimed in claim 4, characterized in that the reaction is carried out at 80–130 °C.

6. The process as claimed in any one of the preceding claims in which a brominating agent is used, characterized in that this latter is bromine.

7. The process as claimed in any one of the preceding claims, characterized in that the metal zinc is used in a catalytic amount.

8. The process as claimed in any one of the preceding claims, characterized in that the chlorinating or brominating agent and the orthoester (IV) are used respectively in an amount of at least one molar equivalent and at least two molar equivalents with respect to the propiophenones (II), the alcohol (III) being used in an amount of at least one equivalent with respect to the propiophenones (II).

9. The process as claimed in any one of the preceding claims, characterized in that in the formulae (I) to (IV), R and R′ representa normal lower alkyl group and Ar a group chosen from the following:

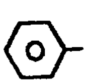 , 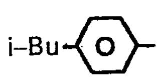 , 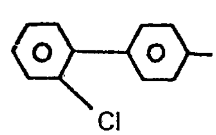 , 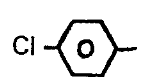 , 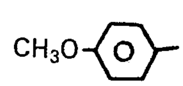

10. As novel industrial product, the compound of formula:

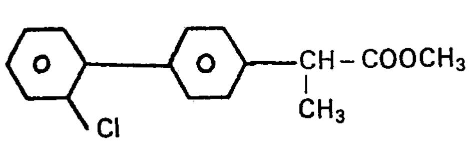 (I′a)

**Claims for the contracting state: AT**

1. A process for preparing hydratropic acids and the esters thereof, corresponding to the formula:

$$Ar{-}\underset{\displaystyle CH_3}{\underset{|}{CH}}{-}COOR \qquad (I)$$

in which:

– R represents a hydrogen atom or a lower alkyl group, and

– Ar represents a phenyl nucleus or a phenyl nucleus substituted by at least one group not likely to de-activate the phenyl nucleus, characterized in that it consists in reacting on the propiophenones of formula:

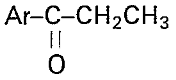 (II)

wherein Ar has the same meaning as in formula (I), a chlorinating or borminating agent, a primary alcohol of formula:

$R'-OH$ (III)

wherein R' represents a lower alkyl group, on orthoester of formula:

$R_1-C(OR')_3$ (IV)

wherein $R_1$ represents a hydrogen atom or a lower alkyl group and R' has the same meaning as in formula (III) and metal zinc, then possibly hydrolyzing the resulting esters of formula:

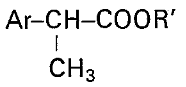 (Ia)

wherein R' has the same meaning as in formula (III) or (IV), to obtain the corresponding acids of formula:

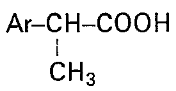 (Ib)

2. The process as claimed in claim 1, characterized in that it consists in reacting the chlorinating or brominating agent on the proopiophenones (II), then in reacting on the resulting compounds, simultaneously the alcohol (III), the orthoester (IV) and the metal zinc.

3. The process as claimed in claim 1, characterized in that it consists in reacting simultaneously on the propiophenones (II), the chlorinating or brominating agent, the alcohol (II), the orthoester (IV) and the metal zinc.

4. The process as claimed in claim 1, 2 or 3, characterized in that the simultaneous reaction of the chlorinating or brominating agent, of the alcohol (III), of the orthoester (IV) and of the metal zinc on the propiophenones (II) or the simultaneous reaction of the alcohol (III), of the orthoester (IV) and of the metal zinc on the reaction product of the chlorinating or brominating agent and the propiophenones is carried out under heating.

5. The process as claimed in claim 4, characterized in that the reaction is carried out at 80–130 °C.

6. The process as claimed in any one of the preceding claims in which a brominating agent is used, characterized in that this latter is bromine.

7. The process as claimed in any one of the preceding claims, characterized in that the metal zinc is used in a catalytic amount.

8. The process as claimed in any one of the preceding claims, characterized in that the chlorinating or brominating agent and the orthoester (IV) are used respectively in an amount of at least one molar equivalent and at least two molar equivalents with respect to the propiophenones (II), the alcohol (III) being used in an amount of at least one equivalent with respect to the propiophenones (II).

9. The process as claimed in any of the preceding claims, characterized in that in the formulae (I) to (IV), R and R' represent a normal lower alkyl group and Ar a group chosen from the following:

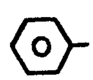 , 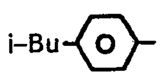 , 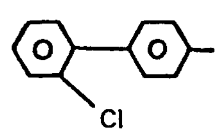 , 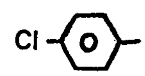 ,